# EUROPÄISCHE PATENTSCHRIFT

(11) **EP 1 574 196 B1**
(45) Veröffentlichungstag und Bekanntmachung des Hinweises auf die Patenterteilung: **07.06.2006**
(21) Anmeldenummer: 04006002.2
(22) Anmeldetag: 12.03.2004
(51) Int. Cl.: A61G 12/00

(54) **Medizinischer Gerätewagen mit einer Andockvorrichtung**
Medical trolley with docking means
Chariot medical avec système d'accrochage

(43) Veröffentlichungstag der Anmeldung: 14.09.2005
(73) Patentinhaber: TRUMPF Kreuzer Medizin Systeme GmbH + Co. KG, 82178 Puchheim (DE)
(72) Erfinder: Schubert, Werner, 88138 Weissenberg (DE); Marka, Rudolf, Dr., 81479 München (DE); Müller, Peter, 85655 Grosshelfendorf (DE)
(74) Vertreter: Hofer, Dorothea

(56) Entgegenhaltungen:
- EP-A- 1 155 673
- WO-A-00/09061
- DE-U- 20 118 251

## Beschreibung

Die Erfindung betrifft einen medizinischen Gerätewagen, insbesondere eine Andockvorrichtung eines fahrbaren medizinischen Gerätewagens an ein Krankenbett. Es kommt häufig vor, daß ein Patient auf einer Intensivstation in einem Krankenhaus an medizinische Geräte angeschlossen oder mit Infusionen versorgt werden muß. Die dazu notwendigen Geräte werden in der Regel auf einem Geräteträger gelagert, der beispielsweise mit einem Wand- oder Deckenstativ gekoppelt ist. Um den Patienten beispielsweise während eines innerklinischen Transportes weiter zu versorgen, werden diese Geräte von dem Wand- oder Deckenstativ an einen fahrbaren Gerätewagen übergeben, der mit einem Krankenbett gekoppelt und gemeinsam damit an den gewünschten Ort bewegt wird.

Aus der DE 201 18 251 U1 ist ein medizinischer Gerätewagen mit einer Ankopplungsvorrichtung für ein Krankenhausbett bekannt. Die Ankopplungsvorrichtung weist einen Schlitten auf, der entlang einem Ständerteil des Gerätewagens in vertikaler Richtung manuell verschiebbar ist. An dem verschiebbaren Schlitten sind Ankopplungselemente zum Ankoppeln an das Krankenbett vorgesehen und der Schlitten selbst ist mittels einer Arretiervorrichtung in der Höhe fixierbar. Durch diese Vorrichtung der DE 201 18 251 U1 wird eine verdrehfeste und lagestabile Einheit des Krankenbetts und des daran angekoppelten Gerätewagens erzielt.

Aus der DE 40 38 427 A1 ist eine Vorrichtung zur lösbaren Verbindung eines fahrbaren medizinischen Gerätewagens mit einem Krankenbett offenbart. Der fahrbare medizinische Gerätewagen besitzt einen Grundkörper mit einem vertikalen Gestellkörper, der mit einer höhenverstellbaren Geräteträgerplatte versehen ist. Am Gestellträger sind verstellbare Halteklauen angeordnet, in die nach unten offene Haken eingesteckt sind, welche ein schnelles Verbinden und Lösen des fahrbaren medizinischen Gerätewagens durch Ein- und Aushängen an Krankenbetten unterschiedlicher Konstruktion gewährleisten. Allerdings ist die Einsetzbarkeit dieser Hakenkonstruktion nicht universell für alle Bettenarten anwendbar, da es unzählige Bettenarten gibt, die unterschiedliche Bügelformen und Abmessungen besitzen, in die die Haken eingehängt werden müssen, so daß jeweils passende Haken dafür vorgesehen sein müssen.

Es ist daher Aufgabe der Erfindung, eine Andockvorrichtung eines fahrbaren medizinischen Gerätewagens bereitzustellen, die universell für annähernd jedes Krankenbett einsetzbar ist und die für eine zuverlässige, einfache und schnelle Verbindung des Gerätewagens mit dem Krankenbett sorgt.

Diese Aufgabe wird mit den Merkmalen des Anspruchs 1 gelöst. Weitere vorteilhafte Ausgestaltungen der Erfindung sind Gegenstand der Unteransprüche.

Gemäß einer vorteilhaften Weiterbildung kann die Aufnahme eines Auslegers des Gerätewagens so ausgebildet sein, daß sie formschlüssig mit einer Stoßschutzvorrichtung des Krankenbettes in Eingriff gelangen kann, indem die Aufnahme von oben oder von unten oder von der Seite durch die Vorspannung des Auslegers gegen die Stoßschutzvorrichtung oder andere Teile des Bettes, z.B. Abschlussplatten, Bügel oder Bettrahmen gedrängt wird.

Das Vorsehen eines Kraftspeichers beispielsweise in Form einer Gasfeder zur Bereitstellung der Vorspannung des Auslegers spart Platz, da eine Gasfeder wenig Bauraum benötigt, und sorgt für einen dauerhaft zuverlässigen Betrieb.

Das Vorsehen eines Handgriffs oder eines Fußhebels an dem beweglichen Ausleger erleichtert die Handhabung der Andockvorrichtung erheblich, indem die Andockvorrichtung einfach entgegen ihrer Vorspannung bewegt wird, bevor sie an das Krankenbett angedockt wird. Befindet sich die Andockvorrichtung in der richtigen Position, in der sich die Aufnahmen unter- oder oberhalb oder seitlich von den Stoßschutzvorrichtungen oder seitlich von den anderen Teilen des Bettes befinden, wird der Handgriff bzw. der Fußhebel losgelassen, wodurch sich der Ausleger automatisch in Richtung der Stoßschutzvorrichtung oder des Teiles des Bettes, in den der Ausleger eingreift bewegt und ein Andockvorgang erfolgt.

Weitere Merkmale und Vorteile der Erfindung ergeben sich aus der Beschreibung eines derzeit bevorzugten Ausführungsbeispiels unter Bezugnahme auf die beigefügten Figuren.
- Fig. 1: ist eine perspektivische Ansicht eines fahrbaren Gerätewagens und eines Krankenbettes.
- Fig. 2: ist eine Seitenansicht eines fahrbaren medizinischen Gerätewagens in der Grundposition.
- Fig. 3: ist eine Seitenansicht des medizinischen Gerätewagens vor dem Andockvorgang.
- Fig. 4: ist eine Seitenansicht des medizinischen Gerätewagens nach dem Andockvorgang.
- Fig. 5: ist eine Draufsicht des medizinischen Gerätewagens im angedockten Zustand.
- Fig. 6: ist eine perspektivische Ansicht der Andockvorrichtung.
- Fig. 7: ist eine perspektivische Ansicht der Andockvorrichtung aus Fig. 6 im zusammenmontierten Zustand mit einem Teil des Gerätewagens.
- Fig. 8: ist eine Draufsicht aus Fig. 7.

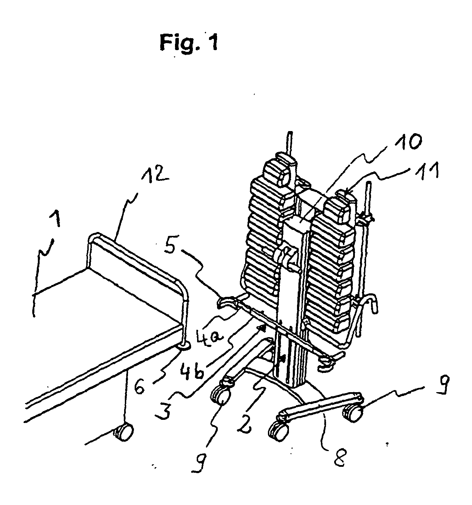

Fig. 1 zeigt einen Teil eines Krankenbettes 1 und einen fahrbaren medizinischen Gerätewagen 2. Der Gerätewagen 2 weist ein Fahrgestell 8 mit vier Rädern 9 auf. Auf dem Fahrgestell 8 ist eine vertikale Säule 10 befestigt. An die Säule 10 ist im vorliegenden Ausführungsbeispiel ein Pumpenstangenträger 11 gekoppelt, der zur Aufnahme von beispielsweise medizinischen Infusionspumpen, etc., dient. Das Krankenbett 1 weist in seiner Längsrichtung eine Abschlußplatte oder einen Bügel 12 auf, der beispielsweise ergriffen wird, um das Bett wegzuschieben. Am unteren Ende des Bügels 12 befindet sich auf jeder Seite eine Stoßschutzvorrichtung 6 in Form von tellerförmigen Bauelementen aus Hartgummi oder Kunststoff.

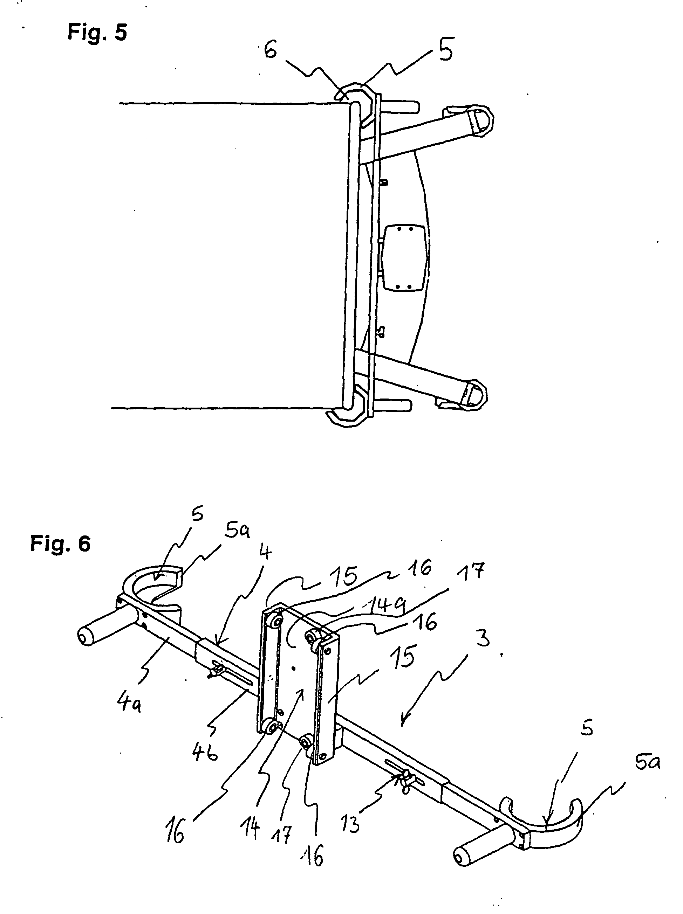

Die Andockvorrichtung 3 wird unter Bezugnahme auf Fig. 6 näher erläutert. Sie weist einen Ausleger 4 in Form eines horizontal verlaufenden Gestänges auf. An jedem Ende des Auslegers 4 ist eine Aufnahme 5 vorgesehen. Im vorliegenden Ausführungsbeispiel besitzt die Aufnahme 5 eine tellerförmige Gestalt, bei der ca. ein Viertelkreis herausgeschnitten ist. Am Umfang der Aufnahme 5 ist eine senkrecht verlaufende Krempe 5a vorgesehen. Der Ausleger 4 besitzt eine längenverstellbare Konstruktion. Dazu weist er auf jeder Seite zwei längliche, ineinander verschiebbare, Elemente auf, im vorliegenden Ausführungsbeispiel eine innere Stange 4a und eine äußere Stange 4b, die so dimensioniert ist, daß die innere Stange 4a in und aus der äußeren Stange 4b geschoben werden kann. Die gleiche Konstruktion befindet sich auch auf der anderen Seite des Auslegers. Es ist eine Arretiervorrichtung 13 vorgesehen, die die Längenverstellung des Auslegers 4 fixiert. Sie besteht im vorliegenden Ausführungsbeispiel aus einem Langloch in jeder Stange 4a, 4b und einer Schraube, die durch die beiden Langlöcher gesteckt wird und dann mit einer Gegenmutter, im vorliegenden Fall einer Flügelmutter, verschraubt wird. Damit kann der Ausleger 4 auf jede beliebige Breite eines Krankenbetts 1 eingestellt werden. In der Mitte des Auslegers 4 ist ein Führungselement 14 angebracht, das sich nach oben erstreckt. Es hat einen C-förmigen Querschnitt mit einer Basisplatte 14a und zwei kurzen Schenkeln 15. Am oberen und unteren Ende der Schenkel 15 sind jeweils Rollen 16 gelagert, deren Achsen parallel zur Basisplatte 14a verlaufen. Auf der Basisplatte sind ebenfalls am oberen und unteren Ende Rollen 17 gelagert, deren Achsen allerdings parallel zu den kurzen Schenkeln 15 verlaufen.

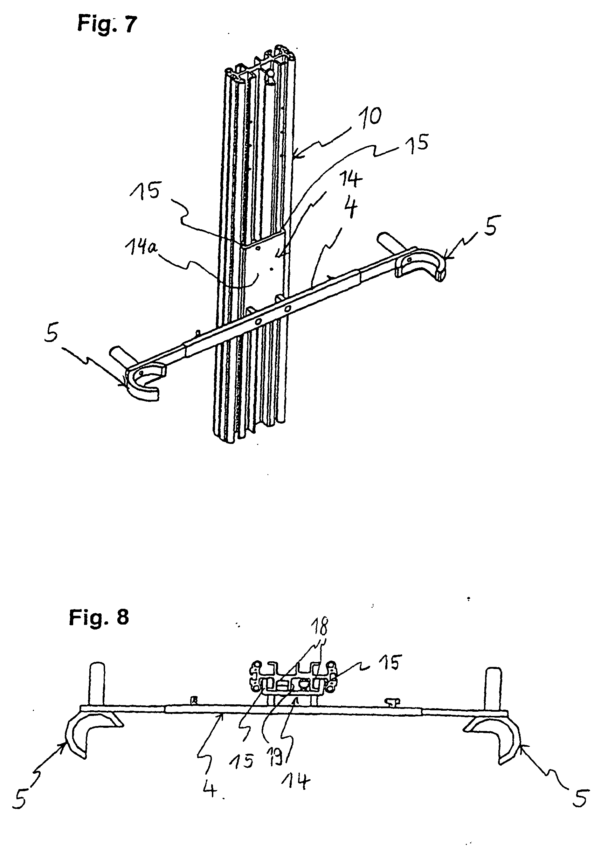

In den Figuren 7 und 8 wird gezeigt, wie das Führungselement 14 in der Säule 10 verschieblich gelagert und durch die Rollen 16, 17 geführt wird. In Fig. 8 ist der Querschnitt der Säule 10 zu erkennen. Die Säule 10 besitzt im wesentlichen einen I-förmigen Profilquerschnitt. Zusätzlich erstrecken sich mehrere Rippen 18 rechtwinklig aus dem Längsabschnitt des I-Profils der Säule. Wie in Fig. 8 gezeigt ist, umgreift das Führungselement 14 mit seinen Schenkeln 15 die rechtwinkligen Rippen 18, so daß die Rollen 15 an der Seite der Säule abrollen. Darüber hinaus sind gerade Rippen 19 an der Seite der Säule vorgesehen, an der die Rollen 17 abrollen. Dadurch wird eine stabile und genaue Führung des Auslegers entlang der Säule 10 erzielt.

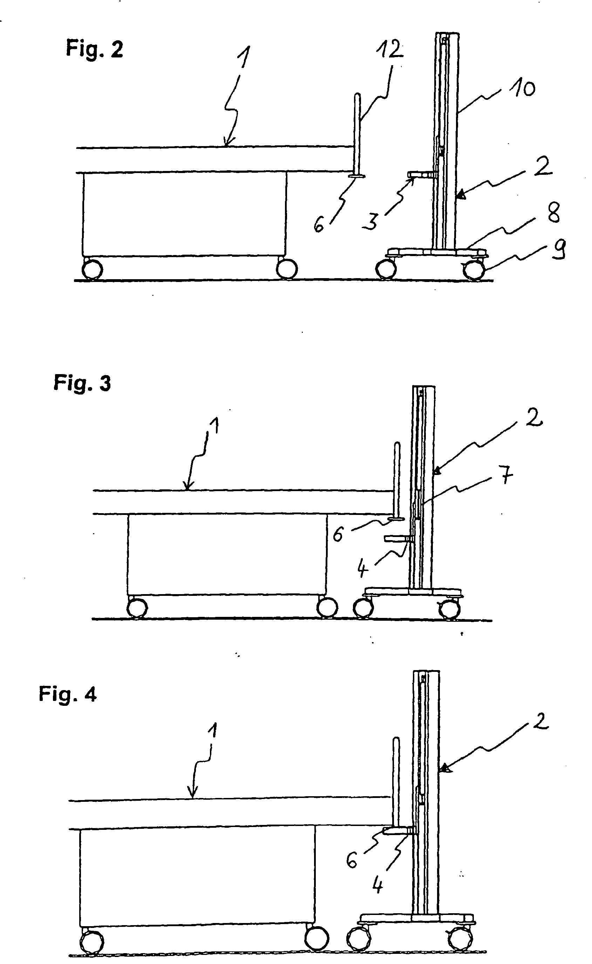

Fig. 2 zeigt den Gerätewagen 2 mit seinem Ausleger 4 in der Grundposition, in der der Ausleger 4 durch die Vorspannung einer nicht gezeigten Gasfeder nach oben in Fig. 1 gedrängt wird. Die oberste Position des Auslegers 4 ist höher als die größte Höhe der Stoßschutzvorrichtung 6 so dass gewährleistet ist, dass beim Überfahren eines Hindernisses durch das Bett die Aufnahmen 5 den Formschluss zu den Stoßschützeinrichtungen behält.

In Fig. 3 befindet sich der Gerätewagen 2 direkt in der Andockposition zu dem Krankenbett 1. In dieser Position wird der Ausleger 4 entgegen der Vorspannkraft der Gasfeder nach unten gedrückt.

Anschließend wird der Ausleger 4 losgelassen, so daß die Vorspannkraft der Gasfeder den Ausleger 4 nach oben in Richtung der Stoßschutzvorrichtung 6 bewegt, bis die Aufnahmen 5 die Stoßschutzvorrichtungen 6 formschlüssig umgreifen, wie in Fig. 4 dargestellt ist.

Wie in den Figuren 7 und 8 gezeigt ist, ist die Aufnahme 5 tellerförmig ausgebildet, wobei ca. ein Viertelkreisabschnitt ausgenommen ist. Dadurch wird das Eingreifen in die Stoßschutzvorrichtungen 6 erleichtert. Der Formschluß und das Umgreifen der Stoßschutzvorrichtung 6 mit der dreiviertelkreisförmigen Krempe 5a sorgt dafür, daß der Gerätewagen 2 fest mit dem Krankenbett 1 verbunden ist. Damit ist ein Bewegen der verblockten Kombination Krankenbett/Gerätewagen möglich, wobei der Gerätewagen sowohl gegen das Krankenbett drückend geschoben, als auch das Krankenbett mitziehend gezogen werden kann.

Die Vorspannung des Auslegers 4 nach oben mittels der nicht dargestellten Gasdruckfeder sorgt für eine sichere Verbindung, selbst bei einer Höhenverstellung des Krankenbettes 1 oder beim Überfahren von Schwellen oder ähnlichem mit dem Krankenbett 1 oder dem Gerätewagen 2, da die Andockvorrichtung 3 durch die Federkraft immer nach oben gedrückt wird und die Aufnahmen 5 immer die Stoßschutzeinrichtungen 6 sicher umschließen.

Das Entkoppeln erfolgt auf einfache Art und Weise durch manuelle Abwärtsbewegung des Auslegers 4 und einem Entfernen des Gerätewagens 2 oder des Krankenbettes 1.

Eine alternative Ausführungsform sieht vor, den Ausleger so zu gestalten, dass er in vertikaler Richtung nur soweit an der Säule beweglich angebracht ist, dass er den Hubbewegungen des Bettes folgen kann und beim Überfahren von Schwellen oder Hindernissen eine vertikale Relativbewegung des Wagens zum Bett zulässt. Eine Grundposition, die eine Bewegung nach oben und nach unten zulässt, wird durch den Einsatz von federnden Elementen erreicht. In der alternativen Ausführungsform ist der Ausleger an sich in seiner Länge veränderbar gestaltet und beispielsweise durch eine Druck- oder Zugfeder so vorgespannt, dass die Aufnahmen 5, die an jedem Ende des Auslegers vorgesehen sind, in eine Richtung gedrängt werden, in der sie aufeinander zu bewegt werden. Die Aufnahmen 5 sind dann so gestaltet, dass sie die Stoßschutzvorrichtungen oder andere Teile des Bettes im angedockten Zustand von der Seite her umfassen und fixieren.

## Patentansprüche

1. Medizinischer Gerätewagen (2) mit einer Andockvorrichtung (3) zum Andocken an ein Krankenbett (1), die einen vertikal bewegbaren Ausleger (4) aufweist, **dadurch gekennzeichnet, dass** der Ausleger vertikal vorgespannt am Gerätewagen (2) angebracht ist, so daß er im angedockten Zustand von oben oder von unten gegen einen Teil des Krankenbetts gedrückt wird ; oder dass der Ausleger horizontal in seiner Länge verstellbar ist und horizontal vorgespannt am Gerätewagen (2) angebracht ist, so daß es im angedockten Zustand von der Seite gegen einen Teil des Krankenbettes gedrückt wird.

2. Medizinischer Gerätewagen (2) nach Anspruch 1, wobei der Ausleger (4) an seinen Enden Aufnahmen (5) aufweist, die dazu angepaßt sind, mit Bauteilen des Krankenbettes (1) in Eingriff zu gelangen.

3. Medizinischer Gerätewagen (2) nach dem vorhergehenden Anspruch, wobei die Aufnahme (5) so ausgebildet ist, daß sie formschlüssig mit einer Stoßschutzvorrichtung (6) des Krankenbettes (1) in Eingriff gelangen kann.

4. Medizinischer Gerätewagen (2) nach einem der vorhergehenden Ansprüche, wobei der Ausleger (4), der vertikal vorgespannt am Gerätewagen (2) angebracht ist in seiner Länge verstellbar ist.

5. Medizinischer Gerätewagen (2) nach einem der vorhergehenden Ansprüche, wobei ein Kraftspeicher (7) am Gerätewagen (2) und am Ausleger (4) so befestigt ist, daß er letzteren in vertikaler oder horizontaler Richtung vorspannt.

6. Medizinischer Gerätewagen (2) nach einem der vorhergehenden Ansprüche, wobei ein Handgriff oder ein Fußhebel an dem Ausleger (4) vorgesehen ist.

## Claims

1. A mobile medical equipment cart (2) with a docking device (3) for docking to a hospital bed (1), said docking device (3) comprising a vertically movable arm (4), **characterized in that** the arm is attached to the equipment cart (2) in a vertically biased manner, such that it is pressed against a part of the hospital bed from above or from below, when it is in its docked position; or that the arm is horizontally adjustable in its length, and attached to the equipment cart (2) in a horizontally biased manner, such that it is pressed against a part of the hospital bed in its docked position.

2. A mobile medical equipment cart (2) according to Claim 1, wherein the arm (4) comprises at its ends receptacles (5) that are adapted to be brought into engagement with members of the hospital bed (1).

3. A mobile medical equipment cart (2) according to the preceding claim, wherein the receptacle (5) is designed such that it can be brought into positive engagement with a collision protection device (6) of the hospital bed (1).

4. A mobile medical equipment cart (2) according to any of the preceding claims, wherein the arm (4) which is attached in a vertically biased manner to the equipment cart (2) is adjustable in its length.

5. A mobile medical equipment cart (2) according to any of the preceding claims, wherein an energy storing device (7) is attached to the equipment cart (2) and to the arm (4) such that it biases the latter in vertical or horizontal direction.

6. A mobile medical equipment cart (2) according to any of the preceding claims, wherein a handle or a foot lever is provided on the arm (4).

## Revendications

1. Chariot médical (2) avec un dispositif d'accrochage (3) pour l'accrochage à un lit de malade (1), présentant un bras articulé (4) déplaçable verticalement, **caractérisé en ce que** le bars articulé est monté sur le chariot (2) de façon pré-contrainte verticalement, de sorte que, à l'état accroché, il soit pressé par le haut ou par le bas contre une partie du lit de malade ; ou en ce que le bras articulé est pré-réglable horizontalement quant à sa longueur et est monté de façon pré-contraint horizontalement sur le chariot (2), de manière que, à l'état accroché, il soit pressé depuis le côté contre une partie du lit de malade.

2. Chariot médical (2) selon la revendication 1, le bras articulé (4) présentant à ses extrémités des logements (5) adaptés pour venir en prise avec des composants du lit de malade (1).

3. Chariot médical (2) selon la revendication précédente, le logement (5) étant configuré de manière à pouvoir venir en prise, par liaison à ajustement de forme, avec un dispositif de protection des chocs (6) du lit de malade (1).

4. Chariot médical (2) selon l'une des revendications précédentes, **caractérisé en ce que** le bras articulé (4), qui est monté de façon pré-contraint verticalement sur le chariot (2), est réglable en longueur.

5. Chariot médical (2) selon l'une des revendications précédentes, un accumulateur de force (6) étant fixé sur le chariot (2) et sur le bras articulé (4), de manière à pré-contraindre ce dernier en direction verticale ou horizontale.

6. Chariot médical (2) selon l'une des revendications précédentes, une manette ou une pédale étant prévue sur le bras articulé (4).
